(19)

Europäisches
Patentamt
European
Patent Office
Office européen
des brevets

(11)     **EP 4 478 034 A1**

(12)     **EUROPEAN PATENT APPLICATION**
published in accordance with Art. 153(4) EPC

(43) Date of publication:
**18.12.2024   Bulletin 2024/51**

(51) International Patent Classification (IPC):
**G01N 21/41** *(2006.01)*      **G01B 11/04** *(2006.01)*

(21) Application number: **23795901.0**

(22) Date of filing: **01.03.2023**

(52) Cooperative Patent Classification (CPC):
**G01B 11/04; G01N 21/41**

(86) International application number:
**PCT/JP2023/007615**

(87) International publication number:
**WO 2023/210153 (02.11.2023 Gazette 2023/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **27.04.2022   JP 2022073082**

(71) Applicant: **Hamamatsu Photonics K.K.
Hamamatsu-shi, Shizuoka 435-8558 (JP)**

(72) Inventors:
• **TSUCHIYA, Kunihiko
  Hamamatsu-shi, Shizuoka 435-8558 (JP)**
• **MATSUMOTO, Toru
  Hamamatsu-shi, Shizuoka 435-8558 (JP)**
• **OHTSUKA, Kenichi
  Hamamatsu-shi, Shizuoka 435-8558 (JP)**

(74) Representative: **Grünecker Patent- und
Rechtsanwälte
PartG mbB
Leopoldstraße 4
80802 München (DE)**

(54)     **REFRACTIVE INDEX DISTRIBUTION MEASUREMENT DEVICE, FILM THICKNESS
DISTRIBUTION MEASUREMENT DEVICE, REFRACTIVE INDEX DISTRIBUTION
MEASUREMENT METHOD, AND FILM THICKNESS DISTRIBUTION MEASUREMENT METHOD**

(57)     A measurement device 1A includes a conveyor 10; a light intensity acquisition unit 20; and an arithmetic processor 30. The conveyor 10 conveys a polymer film in a conveying direction D1. The light intensity acquisition unit 20 irradiates a plurality of spots on the polymer film B being conveyed with light, the plurality of spots being arranged in a direction intersecting the conveying direction D1, and acquires a light intensity of reflected light from each of the plurality of spots irradiated with the light. The arithmetic processor 30 calculates a reflectance at each of the plurality of spots from the light intensity of the reflected light acquired by the light intensity acquisition unit 20, and obtains a refractive index distribution of the polymer film B based on the reflectance.

**Fig.1**

EP 4 478 034 A1

## Description

### Technical Field

The present disclosure relates to a refractive index distribution measurement device, a film thickness distribution measurement device, a refractive index distribution measurement method, and a film thickness distribution measurement method.

### Background Art

Patent Literature 1 and Patent Literature 2 disclose a film thickness measurement device using spectral interferometry. In Patent Literature 1, a method in which a period of the intensity of interference light is obtained using a curve fitting method and a film thickness is calculated based on the period and a refractive index of an object is provided as an example. In Patent Literature 2, a method in which a period of the intensity of interference light is obtained using fast Fourier transform (FFT) and a film thickness is calculated based on the period and a refractive index of an object is provided as an example.

### Citation List

### Patent Literature

Patent Literature 1: Japanese Unexamined Patent Publication No. 2015-141176
Patent Literature 2: International Publication WO 2011/045967

### Summary of Invention

### Technical Problem

When a polymer film is manufactured, a material for the polymer film is formed into a film shape by being stretched in a direction intersecting a conveying direction while the material is conveyed. As a result, a refractive index of the polymer film after being stretched has a variation in the direction intersecting the conveying direction. For example, the refractive index of a peripheral edge portion of the polymer film in the direction intersecting the conveying direction may differ from the refractive index of a central portion of the polymer film in the direction. Such a phenomenon is referred to as the bowing phenomenon. A variation in the refractive index of the polymer film in the direction intersecting the conveying direction leads to a variation in the properties of the polymer film, and causes a measurement error in the measurement of a film thickness or the like. Therefore, it is desirable to measure the variation in the refractive index of the polymer film in the direction intersecting the conveying direction, while the polymer film is conveyed.

An object of the present disclosure is to provide a refractive index distribution measurement device, a film thickness distribution measurement device, a refractive index distribution measurement method, and a film thickness distribution measurement method capable of measuring a variation in a refractive index of a polymer film in a direction intersecting a conveying direction, while the polymer film is conveyed.

### Solution to Problem

[1] A refractive index distribution measurement device according to one embodiment of the present disclosure includes a conveyor, a light intensity acquisition unit and an arithmetic processor. The conveyor conveys a polymer film in a first direction. The light intensity acquisition unit irradiates a plurality of spots on the polymer film being conveyed with light, the plurality of spots being arranged in a second direction intersecting the first direction, and acquires a light intensity of reflected light from each of the plurality of spots irradiated with the light. The arithmetic processor calculates a reflectance at each of the plurality of spots from the light intensity of the reflected light acquired by the light intensity acquisition unit, and obtains a refractive index distribution of the polymer film in the second direction based on the reflectance.

[2] A refractive index distribution measurement method according to one embodiment of the present disclosure includes a step of starting to convey a polymer film in a first direction; a step of irradiating a plurality of spots on the polymer film being conveyed with light, the plurality of spots being arranged in a second direction intersecting the first direction, and acquiring a light intensity of reflected light from each of the plurality of spots irradiated with the light; and a step of calculating a reflectance at each of the plurality of spots from the light intensity of the reflected light acquired in the step of acquiring, and obtaining a refractive index distribution of the polymer film in the second direction based on the reflectance.

When an object receives light and reflects the light, a reflectance of the object depends on a refractive index of the object. In the device and the method, the polymer film being conveyed is irradiated with the light, the reflectance is calculated from the light intensity of the reflected light, and the refractive index of the polymer film is obtained based on the reflectance. Therefore, the refractive index of the polymer film being conveyed can be measured. Furthermore, in the device and the method, such refractive index measurement is performed on the plurality of spots arranged in the direction intersecting the conveying direction. Accordingly, a variation in the refractive index of the polymer film in the direction intersecting the conveying direction can be measured while the polymer film is conveyed.

[3] In the refractive index distribution measurement device according to [1], the arithmetic processor may calculate the reflectance at each of the plurality of spots based on a ratio of the light intensity of the reflected light from each of the plurality of spots to a reference light intensity. The reference light intensity is obtained by irradiating a reference reflective surface, of which a reflectance is known, with the light from the light intensity acquisition unit, and acquiring a light intensity of reflected light from the reference reflective surface in the light intensity acquisition unit. Similarly, in the refractive index distribution measurement method according to [2], in the step of obtaining the refractive index distribution, the reflectance at each of the plurality of spots may be calculated based on a ratio of the light intensity of the reflected light from each of the plurality of spots to a reference light intensity. The reference light intensity is obtained by irradiating a reference reflective surface, of which a reflectance is known, with the light, and acquiring a light intensity of reflected light from the reference reflective surface. Generally, the reflectance is given by the ratio of an intensity of reflected light to an intensity of incident light. By irradiating the reference reflective surface with the light and acquiring the light intensity of the reflected light from the reference reflective surface, the intensity of the incident light can be known with high accuracy. Therefore, in this case, for example, even when the light intensity of the light output from the light intensity acquisition unit, namely, the intensity of the incident light varies over time, the refractive index distribution can be measured with high accuracy.

[4] In the refractive index distribution measurement device according to [3], the light intensity acquisition unit may include the reference reflective surface, and a mechanism for changing a position of the reference reflective surface. The mechanism may change the position of the reference reflective surface between a position on an optical path of the light emitted from the light intensity acquisition unit and a position avoiding the optical path. In this case, the above-described reference light intensity can be easily acquired.

[5] In the refractive index distribution measurement device according to [1], [3], or [4], the light intensity acquisition unit may include a plurality of optical units respectively corresponding to the plurality of spots. Each of the plurality of optical units may include a light-emitting portion that emits the light and a light incident portion that receives the reflected light.

[6] Alternatively, in the refractive index distribution measurement device according to [1], [3], or [4], the light intensity acquisition unit may include an optical unit including a light-emitting portion that emits the light, and a light incident portion that receives the reflected light, and a scanning portion that scans the optical unit in a direction intersecting the first direction. For example, with any of these configurations, the plurality of spots can be irradiated with the light, and the light intensity of the reflected light from each of the plurality of spots irradiated with the light can be acquired.

[7] In the refractive index distribution measurement device according to any of [1] and [3] to [6], the arithmetic processor may obtain the refractive index distribution further based on an extinction coefficient of the polymer film. Similarly, in the refractive index distribution measurement method according to [2] or [3], in the step of obtaining the refractive index distribution, the refractive index distribution may be obtained further based on an extinction coefficient of the polymer film. More precisely, a refractive index of an object is also correlated with an extinction coefficient of the object in addition to a reflectance. Therefore, by obtaining the refractive index distribution further based on the extinction coefficient of the polymer film, a variation in the refractive index of the polymer film can be measured with higher accuracy.

[8] In the refractive index distribution measurement device according to any of [1] and [3] to [7] or the refractive index distribution measurement method according to [2], [3], or [7], a wavelength band of the light may be in a visible wavelength range, or may be a wavelength band ranging from the visible wavelength range to a near-infrared wavelength range.

[9] In the refractive index distribution measurement device according to any of [1] and [3] to [7] or the refractive index distribution measurement method according to [2], [3], or [7], a wavelength band of the light may include a near-infrared wavelength range. When the wavelength band of the light includes the near-infrared wavelength range, the light can transmit through the polymer film even when the polymer film has a color. Therefore, the influence of the color of the polymer film on the measurement result can be reduced.

A film thickness distribution measurement device according to one embodiment of the present disclosure includes the refractive index distribution measurement device according to any of [1] and [3] to [9]. The arithmetic processor further obtains a film thickness distribution of the polymer film in the second direction based on the light intensity of the reflected light at each of the plurality of spots acquired by the light intensity acquisition unit and on the refractive index distribution. A film thickness distribution measurement method according to one embodiment of the present disclosure includes the refractive index distribution measurement method according to any of [2], [3], and [7] to [9]; and a step of obtaining a film thickness distribution of the polymer film in the second direction based on the light intensity of the reflected light at each of the plurality of spots acquired in the step of acquiring the light intensity and on the refractive index distribution obtained in the step of obtaining the refractive index distribution. According to the film thickness distribution measurement device and the film thickness distribution measurement method, while the polymer film is conveyed, the film thickness distribution of the polymer film can be measured with high accuracy while taking into consideration a variation in the refractive index.

## Advantageous Effects of Invention

According to the present disclosure, it is possible to provide the refractive index distribution measurement device, the film thickness distribution measurement device, the refractive index distribution measurement method, and the film thickness distribution measurement method capable of measuring a variation in the refractive index of the polymer film in the direction intersecting the conveying direction, while the polymer film is conveyed.

## Brief Description of Drawings

[FIG. 1] FIG. 1 is a view schematically illustrating a configuration of a measurement device according to one embodiment.
[FIG. 2] FIG. 2 is an enlarged side view illustrating an optical unit.
[FIG. 3] FIG. 3 is a plan view illustrating the optical unit and a linear guide.
[FIG. 4] Parts (a) and (b) in FIG. 4 are graphs illustrating examples of a relationship between reflectance and wavelength.
[FIG. 5] Parts (a) and (b) in FIG. 5 are graphs illustrating examples of a relationship between reflectance and wavelength.
[FIG. 6] FIG. 6 is a view for describing the principle of film thickness measurement.
[FIG. 7] Parts (a), (b), and (c) in FIG. 7 are graphs illustrating a relationship between the intensity and the wavelength of reflected light after interference.
[FIG. 8] FIG. 8 is a diagram schematically illustrating a configuration example of hardware of an arithmetic processor.
[FIG. 9] FIG. 9 is a diagram schematically illustrating a configuration of a control device.
[FIG. 10] FIG. 10 is a flowchart illustrating a method for measuring a refractive index distribution and a film thickness distribution according to one embodiment.
[FIG. 11] Parts (a), (b), and (c) in FIG. 11 are front views illustrating a configuration of an optical unit as a first modification example.
[FIG. 12] Parts (a), (b), and (c) in FIG. 12 are front views illustrating a configuration of a light intensity acquisition unit as a second modification example.
[FIG. 13] FIG. 13 is a view schematically illustrating a configuration of a measurement device according to a third modification example.
[FIG. 14] FIG. 14 is a plan view illustrating a plurality of optical units.
[FIG. 15] Parts (a) and (b) in FIG. 15 are front views illustrating a mode in which the plurality of optical units are disposed side by side in a direction intersecting a conveying direction.

## Description of Embodiments

Hereinafter, embodiments of a refractive index distribution measurement device, a film thickness distribution measurement device, a refractive index distribution measurement method, and a film thickness distribution measurement method according to the present disclosure will be described in detail with reference to the accompanying drawings. Incidentally, in the description of the drawings, the same elements are denoted by the same reference signs, and duplicate descriptions will be omitted.

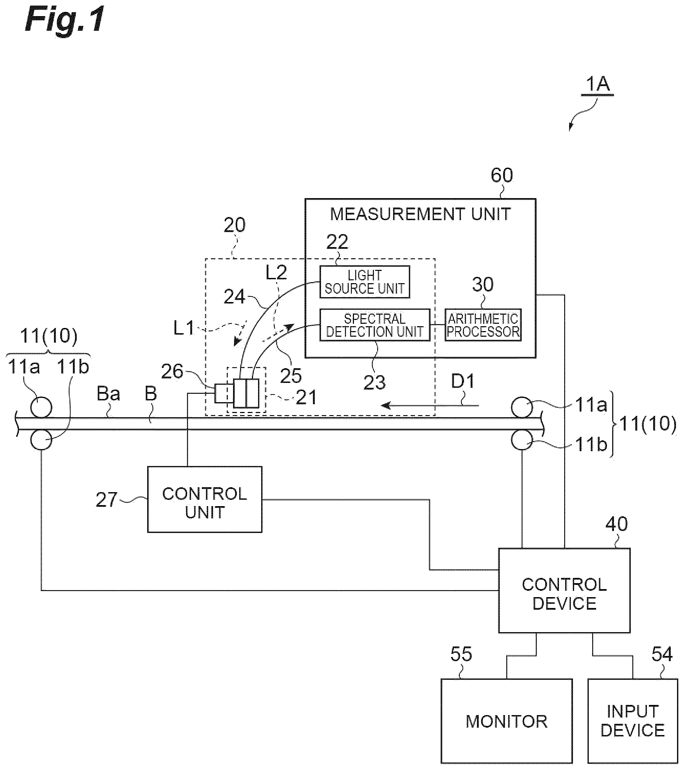

FIG. 1 is a view schematically illustrating a configuration of a measurement device 1A according to one embodiment of the present disclosure. The measurement device 1A is a device that obtains a refractive index distribution and a film thickness distribution of a polymer film B to be measured in a direction intersecting a conveying direction. The polymer film B is, for example, a highly-functional film or a plastic sheet used in a liquid crystal display, a separator used in a lithium ion battery, or the like. The polymer film B of the present embodiment may be composed of a single layer (single material), or may have a structure in which a plurality of layers are laminated. However, when the plurality of layers are laminated, it is assumed that the refractive indexes of the layers other than the layer to be measured are uniform in the direction intersecting the conveying direction and are known.

The measurement device 1A includes a conveyor 10, a light intensity acquisition unit 20, an arithmetic processor 30, a control device 40, an input device 54, and a monitor 55. The conveyor 10 conveys the polymer film B in a conveying direction D1 (first direction). The conveyor 10 is, for example, a roller conveyor, and includes a plurality of roller pairs 11 and a drive unit (not illustrated) such as a motor that rotationally drives the plurality of roller pairs 11. Each of the plurality of roller pairs 11 includes a pair of rollers 11a and 11b having rotation axes extending in a width direction of the polymer film B. The polymer film B is conveyed by rotating the roller 11a and the roller 11b in opposite directions in a state where the polymer film B is sandwiched between the roller 11a and the roller 11b.

The light intensity acquisition unit 20 irradiates a plurality of spots with light, the plurality of spots being arranged in a direction (second direction) intersecting with the conveying direction D1, and acquires a light intensity of reflected light from each of the plurality of spots irradiated with the light. For this purpose, the light intensity acquisition unit 20 includes an optical unit 21, a light source unit 22, a spectral detection unit 23, light guide members 24 and 25, and a linear guide 26.

The light source unit 22 generates non-coherent (incoherent) light L1. A wavelength band of the light L1 may be in the visible wavelength range. In that case, the light source unit 22 is, for example, a lamp type light source, a white LED, or the like that emits white light. The wavelength band of the light L1 may be a wavelength band ranging from the visible wavelength range to the near-infrared wavelength range. The wavelength band of the light L1 may have a substantially flat (broad) spectrum in the infrared wavelength range. Particularly, when the wavelength band of the light L1 includes the near-infrared wavelength range, the light L1 can transmit through the polymer film B even when the polymer film B has a color. Therefore, the influence of the color of the polymer film B on the measurement result can be reduced. In that case, various light-emitting elements such as an amplified spontaneous emission (ASE) light source, an LED, and a super luminescent diode (SLD) can be applied as the light source unit 22. A white light source and an optical component such as an optical film may be combined with each other.

The light guide member 24 is optically coupled to the light source unit 22 at one end thereof, and guides the light L1 emitted from the light source unit 22. For example, a light guide, an optical fiber, or the like is used as the light guide member 24. The optical unit 21 is optically coupled to the other end of the light guide member 24, and irradiates the polymer film B with the light L1 guided by the light guide member 24. Reflected light L2 from the polymer film B is incident on the optical unit 21. The optical unit 21 is disposed at a position facing a major surface Ba of the polymer film B. The light guide member 25 is optically coupled to the optical unit 21 at one end thereof, and guides the reflected light L2 incident on the optical unit 21. For example, a light guide, an optical fiber, or the like is used as the light guide member 25. The spectral detection unit 23 is optically coupled to the other end of the light guide member 25, spectrally separates the reflected light L2 for each wavelength, the reflected light L2 being guided by the light guide member 25, and detects an intensity of the spectrally-separated light for each wavelength. The spectral detection unit 23 is configured, for example, by combining a spectral optical element and an imaging element. The spectral optical element is, for example, a prism or a grating element. The imaging element is, for example, a line sensor, an area image sensor, a photomultiplier tube, or a photodiode. The spectral detection unit 23 outputs the detected light intensity as an electrical signal. The light source unit 22 and the spectral detection unit 23 are accommodated in a housing of a measurement unit 60, together with the arithmetic processor 30 to be described later.

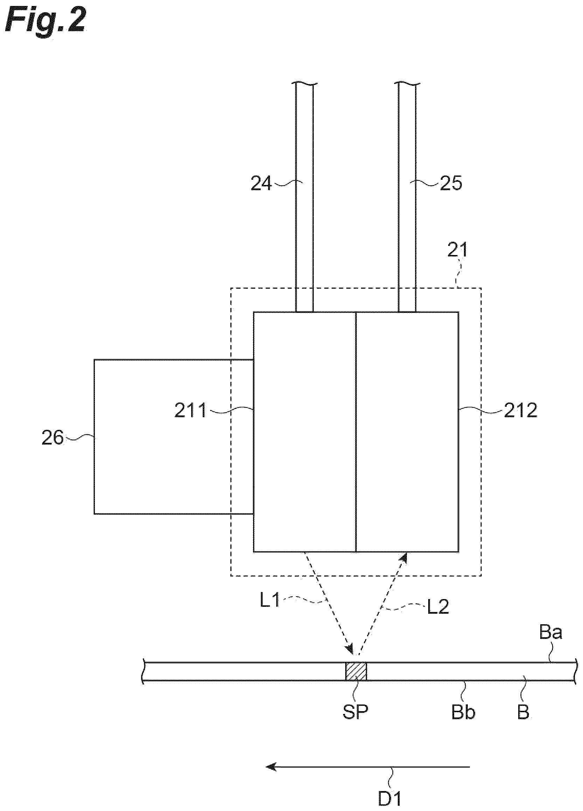

FIG. 2 is an enlarged side view illustrating the optical unit 21. The optical unit 21 includes a light-emitting portion 211 and a light incident portion 212. The light-emitting portion 211 irradiates a spot SP on the polymer film B with the light L1. The light L1 is obtained from the light source unit 22 via the light guide member 24. The light-emitting portion 211 includes, for example, a condenser lens optically coupled to the other end of the light guide member 24. The light incident portion 212 receives the reflected light L2 generated by reflection of the light L1 at the spot SP on the polymer film B. The reflected light L2 includes light reflected by the major surface Ba of the polymer film B, and light reflected by a surface Bb opposite to the major surface Ba. The light incident portion 212 includes, for example, a condenser lens optically coupled to the one end of the light guide member 25. An optical axis of the light-emitting portion 211 and an optical axis of the light incident portion 212 may be parallel to each other, or may intersect each other in the polymer film B. Alternatively, the optical axis of the light-emitting portion 211 and the optical axis of the light incident portion 212 may coincide with each other. The light source unit 22, the light guide member 24, and the light-emitting portion 211 constitute a light irradiation unit that irradiates the polymer film B with the light L1. The light incident portion 212, the light guide member 25, and the spectral detection unit 23 constitute a light detection unit that detects a light intensity of the reflected light L2 from the polymer film B for each wavelength.

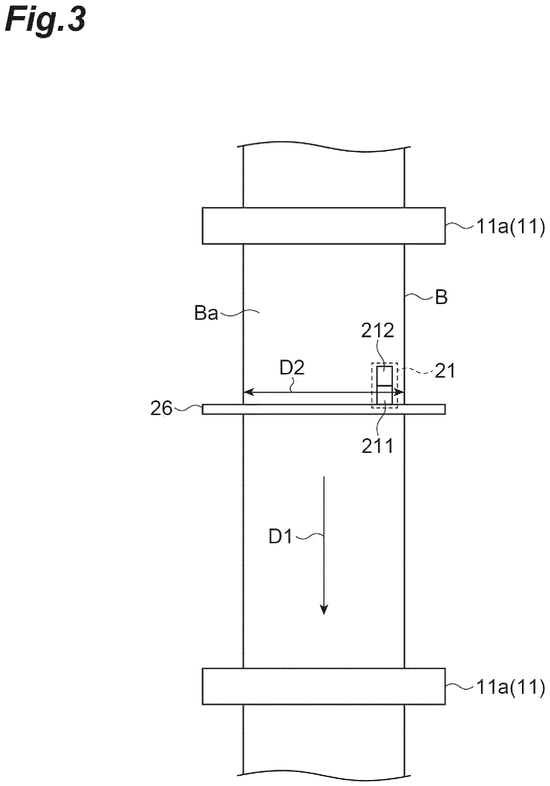

FIG. 3 is a plan view illustrating the optical unit 21 and the linear guide 26. The linear guide 26 is an example of a scanning portion in the present embodiment. The linear guide 26 supports the optical unit 21, and scans the optical unit 21 in a direction D2 intersecting the conveying direction D1. In other words, the linear guide 26 allows the optical unit 21 to move one-dimensionally along the direction D2. In one example, the direction D2 is the width direction of the polymer film B, and is orthogonal to the conveying direction D1. The linear guide 26 is electrically connected to a control unit 27, and is driven by a drive signal provided from the control unit 27. The control unit 27 controls the linear guide 26 such that the optical unit 21 moves back and forth between one end edge and the other end edge of the polymer film B in the direction D2. The linear guide 26 includes, for example, an actuator and a slider stage, and is configured such that the slider stage holding the optical unit 21 is movable back and forth in the direction D2 by the actuator. For example, the actuator of the linear guide 26 is composed of a ball screw including a rotating shaft, and a motor such as a servo motor or a pulse motor, and the ball screw is rotated by the motor, thereby moving the slider stage. In such a manner, the optical unit 21 can irradiate a plurality of the spots SP with the light L1, the plurality of spots SP being arranged in the direction (second direction) intersecting the conveying direction D1, and acquire the light intensity of the reflected light L2 from each of the plurality of spots SP, by performing irradiation with the light L1 and receiving the input of the reflected light L2 while being scanned along the direction D2. In the present embodiment, the direction intersecting with the conveying direction D1 is a direction of a vector obtained by combining a speed vector in the conveying direction D1 and a scanning vector in the direction D2.

Referring again to FIG. 1, the arithmetic processor 30 calculates a reflectance at each of the plurality of spots SP from the light intensity of the reflected light L2 acquired by the light intensity acquisition unit 20. The reflectance is calculated based on the ratio of the light intensity of the reflected light L2 to a reference light intensity. The reference light intensity is typically a light intensity of the light L1. The light intensity of the light L1 is obtained, for example, by irradiating a reference reflective surface, of which the reflectance is known, with the light L1, and acquiring a light intensity of reflected light from the reference reflective surface. In one example, the reference reflective surface is a mirror surface having a reflectance of approximately 100%. Then, the arithmetic processor 30 obtains the refractive index distribution of the polymer film B in the direction intersecting with the conveying direction D1, based on the reflectance. Here, a refractive index n of a certain medium is expressed as shown in Formula (1) below using a reflectance R at an interface between the medium and a surrounding medium.

[Formula 1]

$$ n = \left( \frac{1+R}{1-R} \right) + \sqrt{ \frac{4R}{(1-R)^2} - k^2 } \quad \cdots \quad (1) $$

k in the formula is an extinction coefficient specific to the material. The extinction coefficient k can be obtained in advance from the compositions of the polymer film B. In addition, since the extinction coefficient k is an extremely small numerical value compared to the reflectance R, the extinction coefficient k may be ignored in the above formula. In that case, Formula (1) can be rewritten as Formula (2) below.

[Formula 2]

$$ n = \left( \frac{1+R}{1-R} \right) + \sqrt{ \frac{4R}{(1-R)^2} } \quad \cdots \quad (2) $$

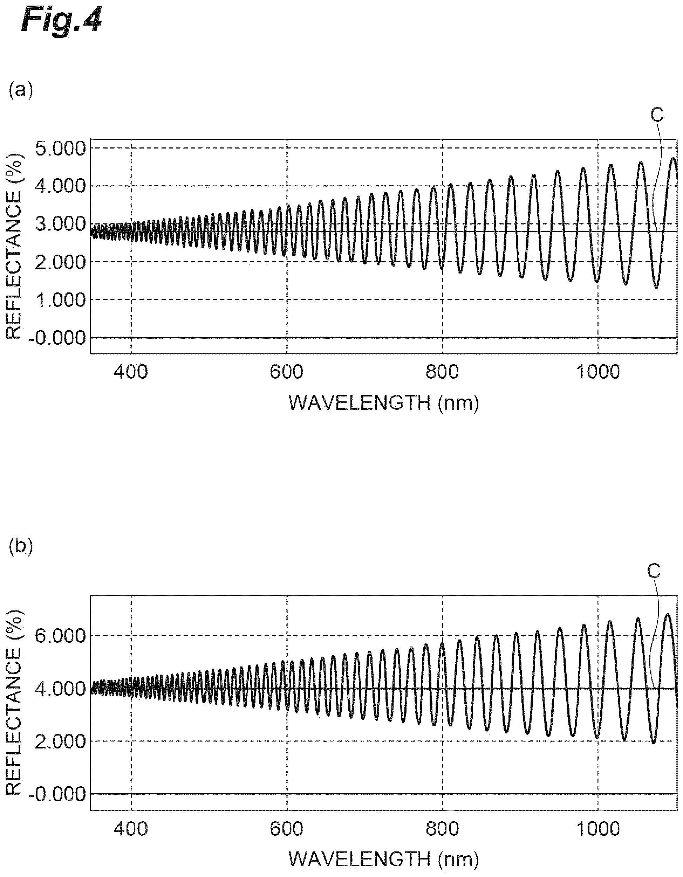

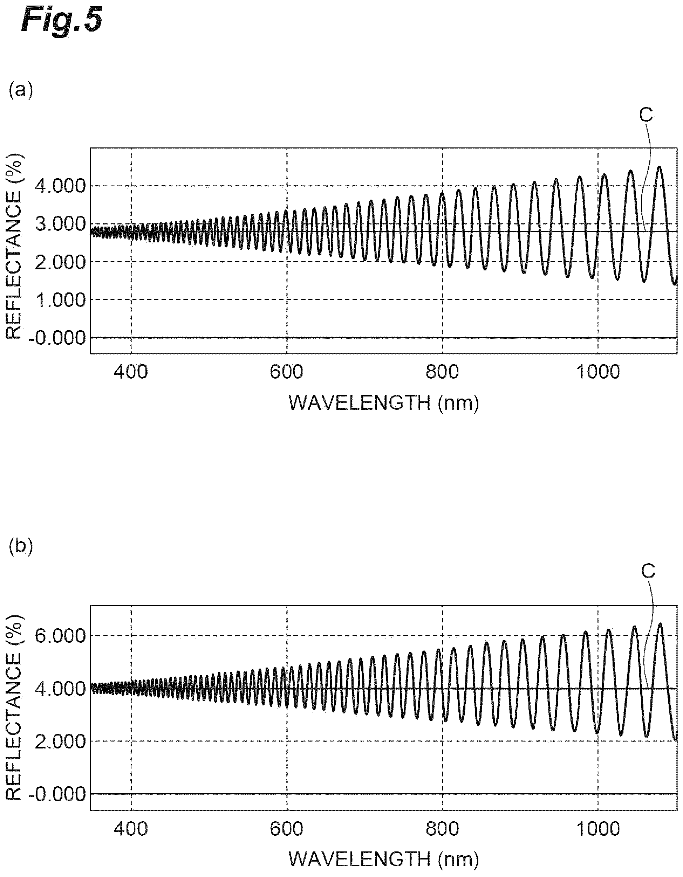

In the present embodiment, the spectral detection unit 23 detects the light intensity of the reflected light L2 for each wavelength. Parts (a) and (b) in FIG. 4 and parts (a) and (b) in FIG. 5 are graphs illustrating examples of a relationship between reflectance and wavelength. In these graphs, the vertical axis represents the reflectance (%) and the horizontal axis represents the wavelength (nm). The extinction coefficient k is 0.01. As described above, the reflected light L2 includes light reflected by the major surface Ba of the polymer film B and light reflected by the surface Bb opposite to the major surface Ba. Since optical path lengths of these lights differ from each other by a thickness of the polymer film B, these lights interfere with each other. Therefore, as illustrated in these graphs, the reflectance varies periodically with the wavelength. As the reflectance R in Formula (1) above, for example, a wavelength average value of the varying reflectance (straight line C in the graphs) is used.

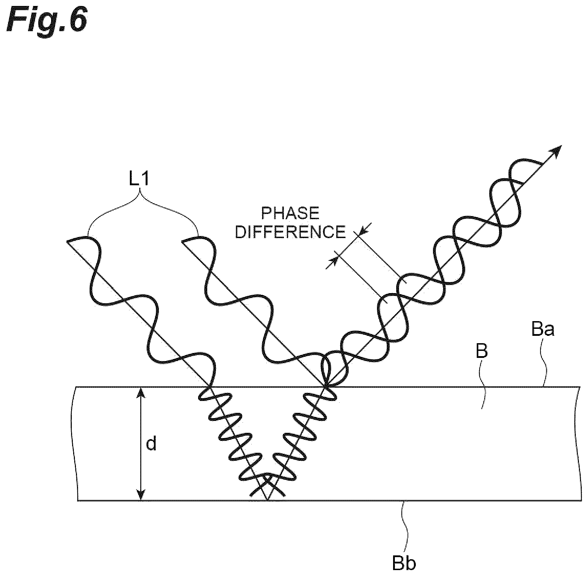

The arithmetic processor 30 further obtains a film thickness distribution of the polymer film B in the direction intersecting the conveying direction D1, based on the light intensity of the reflected light L2 at each of the plurality of spots SP acquired by the light intensity acquisition unit 20 and on the above-described refractive index distribution. FIG. 6 is a view for describing the principle of film thickness measurement, and schematically illustrates a cross section of the polymer film B. In this case, when the incoherent light L1 is incident on the polymer film B, reflected light on the major surface Ba of the polymer film B and reflected light on the surface Bb opposite to the major surface Ba interfere with each other. When the major surface Ba is a light incident surface, an optical path length of the reflected light on the surface Bb is longer than an optical path length of the reflected light on the major surface Ba by the length of an optical path inside the polymer film B. Therefore, a phase difference corresponding to a film thickness d of the polymer film B occurs between these reflected lights.

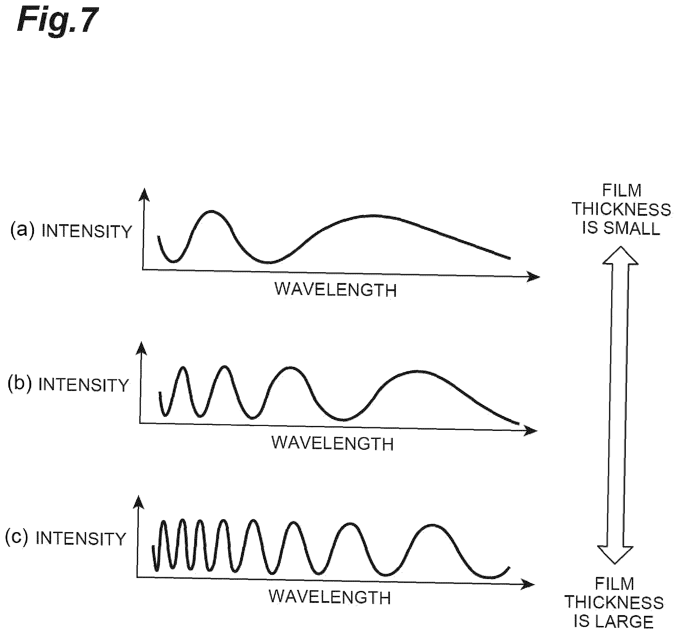

Parts (a), (b), and (c) in FIG. 7 are graphs illustrating a relationship between the intensity and the wavelength of the reflected light after interference. Part (a) in FIG. 7 illustrates a case where the film thickness d of the polymer film B is thinner than that in parts (b) and (c), and part (c) in FIG. 7 illustrates a case where the film thickness d of the polymer film B is thicker than that in parts (a) and (b). As illustrated in FIG. 7, the spectrum of the reflected light after interference (reflection spectrum) undulates due to the interference, and the interval, namely, the period of the waves becomes smaller as the film thickness d of the polymer film B becomes thicker. Namely, the film thickness d of the polymer film B is expressed as shown in Formula (3) below using the refractive index n of the polymer film B, wavelengths $\lambda_1$ and $\lambda_2$ of the light L1, a difference $\Delta m$ between a wavenumber of the reflected light L2 at the wavelength $\lambda_1$ and a wavenumber of the reflected light L2 at the wavelength $\lambda_2$, and an incident angle $\theta$.

[Formula 3]

$$d = \frac{\Delta m}{2\sqrt{n^2 - \sin^2 \theta}} \times \frac{1}{\dfrac{1}{\lambda_2} - \dfrac{1}{\lambda_1}} \quad \cdots \quad (3)$$

The film thickness d of the polymer film B at each of the plurality of spots SP, namely, the film thickness distribution in the direction intersecting the conveying direction D1 can be obtained by using the above-described relationship between the reflection spectrum and the film thickness d of the polymer film B. Specific methods include a fast Fourier transform method and a curve fitting method. The fast Fourier transform method is a method in which a fast Fourier transform is performed on the reflection spectrum and the film thickness d is obtained from a peak frequency thereof. In the curve fitting method, a spectral reflectance obtained from the measured reflection spectrum, namely, a measured spectral reflectance and a theoretical spectral reflectance calculated from a theoretical formula are fitted to each other. Then, the film thickness d is obtained from the fitted theoretical spectral reflectance. According to the curve fitting method, the film thickness distribution can be measured with high accuracy even when the film thickness d of the polymer film B is 1 $\mu$m or less. Data on the film thickness distribution of the polymer film B obtained in this way is provided to the control device 40.

For example, from the data illustrated in part (a) of FIG. 4, the refractive index n is calculated to be 1.4, and the film thickness d is calculated to be 10 $\mu$m. From the data illustrated in part (b) of FIG. 4, the refractive index n is calculated to be 1.5, and the film thickness d is calculated to be 10 $\mu$m. From the data illustrated in part (a) of FIG. 5, the refractive index n is calculated to be 1.4, and the film thickness d is calculated to be 11 $\mu$m. From the data illustrated in part (b) of FIG. 5, the refractive index n is calculated to be 1.5, and the film thickness d is calculated to be 11 $\mu$m.

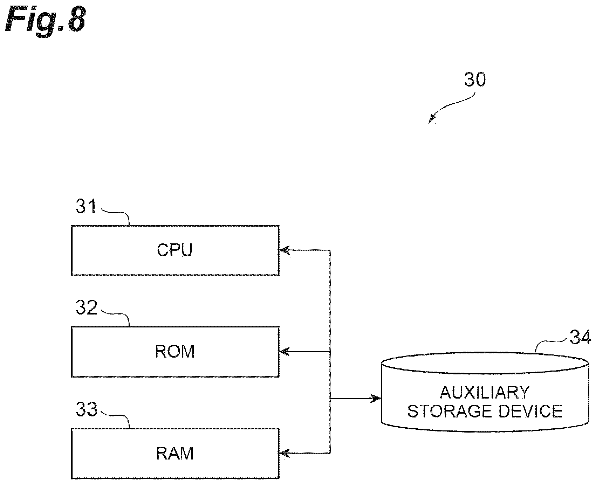

The arithmetic processor 30 may be, for example, a personal computer; a smart device such as a smartphone or a tablet terminal; or a computer including a processor, such as a cloud server. FIG. 8 is a diagram schematically illustrating a configuration example of hardware of the arithmetic processor 30. As illustrated in FIG. 8, the arithmetic processor 30 can be physically configured as a normal computer including a processor (CPU) 31, a main storage device such as a ROM 32 and a RAM 33, an auxiliary storage device 34 such as a hard disk, and the like. The processor 31 of the computer can realize functions of the arithmetic processor 30 by reading out a program stored in the ROM 32 or the auxiliary storage device 34. Therefore, the program causes the processor 31 of the computer to operate as the arithmetic processor 30. The storage device that stores the program may be a non-transitory recording medium. As an example of the recording medium, a recording medium such as a flexible disk, a CD, or a DVD, a recording medium such as a ROM, a semiconductor memory, a cloud server, or the like is provided.

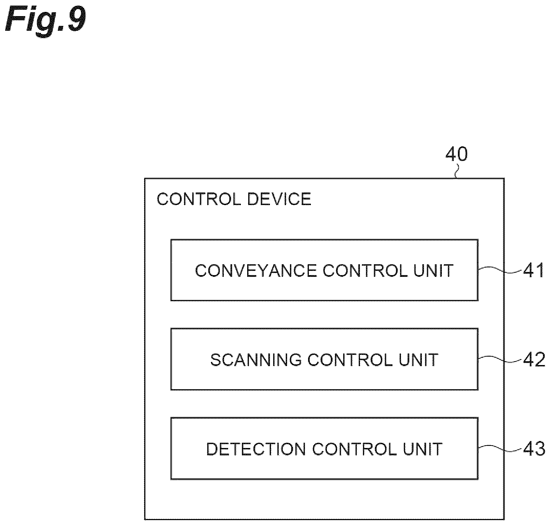

Referring again to FIG. 1, the control device 40 is electrically connected to the conveyor 10, the control unit 27, and the measurement unit 60. FIG. 9 is a diagram schematically illustrating a configuration of the control device 40. The control device 40 includes a conveyance control unit 41 that controls operation of the conveyor 10, such as conveying speed; a scanning control unit 42 that controls operation of the control unit 27; and a detection control unit 43 that controls operation of the light source unit 22 and the spectral detection unit 23. The control device 40 may have, for example, any of the hardware configurations described related to the arithmetic processor 30.

The input device 54 is electrically connected to the control device 40. An operator inputs various settings related to the conveyance control unit 41, the scanning control unit 42, and the detection control unit 43 through the input device 54. The input device 54 can be, for example, a keyboard, a mouse, or a touch panel. The monitor 55 is electrically connected to the control device 40, and displays information on the refractive index distribution and the film thickness distribution obtained by the arithmetic processor 30. The monitor 55 may display only information on the refractive index distribution, or may display only information on the film thickness distribution. The monitor 55 may be a touch screen including the input device 54 that is a touch panel.

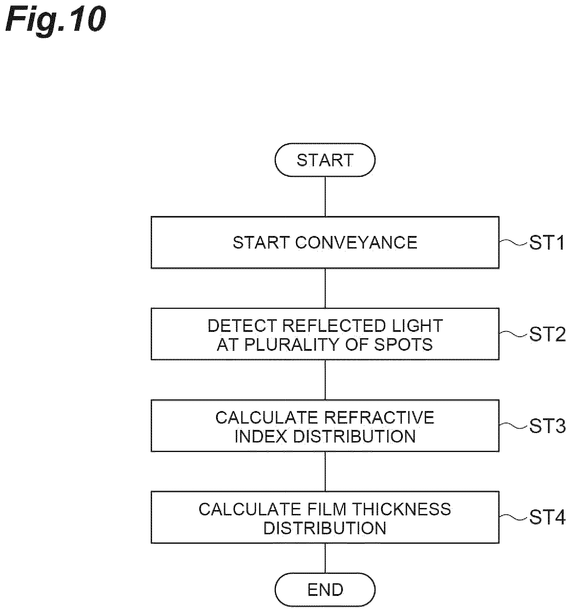

FIG. 10 is a flowchart illustrating a method for measuring a refractive index distribution and a film thickness distribution according to the present embodiment. This measurement method can be performed using the measurement device 1A described above. In this measurement method, in step ST1, the conveyance of the polymer film B in the conveying direction D1 is started. In step ST2, the irradiation of the polymer film B being conveyed with the light L1 and the detection of the reflected light L2 from the polymer film B are performed sequentially on the plurality of spots SP arranged in the direction intersecting the conveying direction D1. In step ST3, the reflectance R at each of the plurality of spots SP is calculated from the light intensity of the reflected light L2 acquired in step ST2. Then, the refractive index distribution of the polymer film B in the direction is calculated based on the reflectance R. In step ST4, the film thickness distribution of the polymer film B in the direction is calculated based on the light intensity of the reflected light L2 at each of the plurality of spots SP acquired in step ST2 and the refractive index distribution calculated in step ST3.

Effects obtained by the measurement device 1A and the measurement method according to the present embodiment described above will be described. When an object receives light and reflects the light, a reflectance of the object depends on a refractive index of the object. In the measurement device 1A and the measurement method of the present embodiment, the polymer film B being conveyed is irradiated with the light L1, the reflectance R is calculated from the light intensity of the reflected light L2, and the refractive index n of the polymer film B is obtained based on the reflectance R. Therefore, the refractive index n of the polymer film B being conveyed can be measured. Furthermore, in the measurement device 1A and the measurement method of the present embodiment, such refractive index measurement is performed on the plurality of spots SP arranged in the direction intersecting the conveying direction D1. Accordingly, a variation in the refractive index n of the polymer film B in the direction intersecting the conveying direction D1 can be measured while the polymer film B is conveyed. In addition, according to the measurement device 1A and the measurement method of the present embodiment, while the polymer film B is conveyed, the film thickness distribution of the polymer film B can be measured with high accuracy while taking into consideration a variation in the refractive index.

As in the present embodiment, the light intensity acquisition unit 20 may include the optical unit 21 including the light-emitting portion 211 that emits the light L1 and the light incident portion 212 that receives the reflected light L2, and the linear guide 26 that scans the optical unit 21 along the direction D2 intersecting the conveying direction D1. For example, with such a configuration, the plurality of spots SP can be irradiated with the light L1, and the light intensity of the reflected light L2 from each of the plurality of spots SP irradiated with the light L1 can be acquired.

As described above, the arithmetic processor 30 may obtain the refractive index distribution further based on the extinction coefficient k of the polymer film B. More precisely, a refractive index of an object is also correlated with an extinction coefficient of the object in addition to a reflectance. Therefore, the arithmetic processor 30 obtains the refractive index distribution further based on the extinction coefficient k of the polymer film B, so that a variation in the refractive index of the polymer film B can be measured with higher accuracy.

[First modification example]

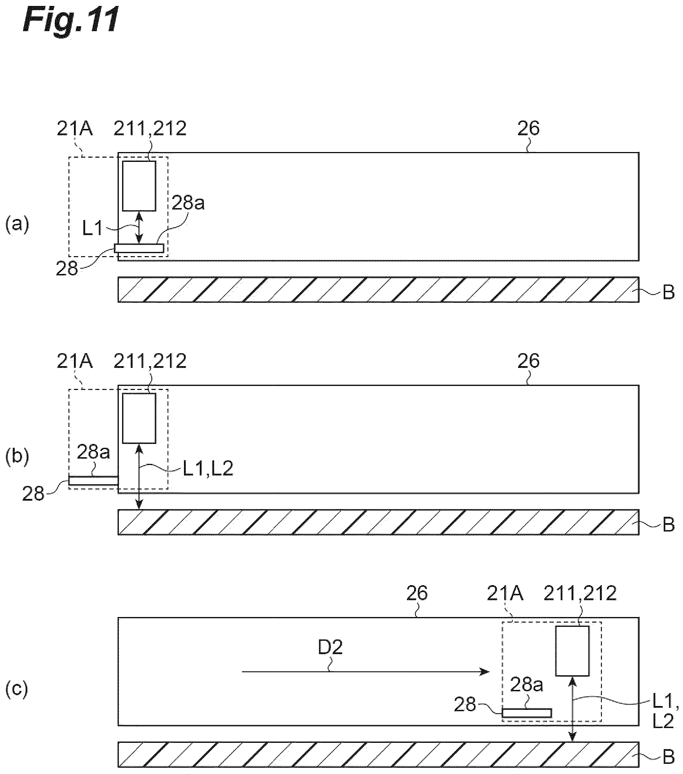

Parts (a), (b), and (c) in FIG. 11 are front views illustrating a configuration of an optical unit 21A as a first modification example. In these figures, the polymer film B is illustrated in a cross section cut along a plane perpendicular to the conveying direction D1. The optical unit 21A of the present modification example further includes a reflective plate 28 in addition to the configuration of the optical unit 21 of the embodiment. The reflective plate 28 has a reference reflective surface 28a of which the reflectance is known. The reflectance of the reference reflective surface 28a is, for example, 100%. The optical unit 21A further includes a mechanism for changing the position of the reference reflective surface 28a between a position on an optical path of the light L1 emitted from the light-emitting portion 211 and a position avoiding the optical path. The operation of the mechanism is controlled by, for example, the control device 40. Part (a) in FIG. 11 illustrates a case where the reference reflective surface 28a is located on the optical path of the light L1 emitted from the light-emitting portion 211. In this case, the light L1 emitted from the light-emitting portion 211 is reflected by the reference reflective surface 28a. Reflected light from the reference reflective surface 28a is incident on the light incident portion 212. Then, the light intensity of the reflected light for each wavelength is detected by the spectral detection unit 23. Accordingly, the arithmetic processor 30 can obtain the reference light intensity, which is to be used when the reflectance R of the polymer film B is calculated, for each wavelength. When the reflectance of the reference reflective surface 28a is 100%, the reference light intensity for each wavelength is equal to a light intensity of the wavelength component of the light L1. Part (b) in FIG. 11 illustrates a case where the reference reflective surface 28a is located to avoid the optical path of the light L1 emitted from the light-emitting portion 211. In this case, the light L1 emitted from the light-emitting portion 211 is reflected by the polymer film B. The reflected light L2 from the polymer film B is incident on the light incident portion 212. The light intensity of the reflected light L2 for each wavelength is detected by the spectral detection unit 23. The arithmetic processor 30 calculates the refractive index n of the polymer film B based on these light intensities. While maintaining the state where the reference reflective surface 28a avoids the optical path of the light L1, as illustrated in part (c) of FIG. 11, the linear guide 26 scans the optical unit 21A along the direction D2 intersecting the conveying direction D1. At this time, the arithmetic processor 30 can obtain the refractive index distribution by repeating the calculation of the refractive index n. The acquisition of the reference light intensity using the reference reflective surface 28a (part (a) in FIG. 11) is repeatedly performed at a predetermined timing. The predetermined timing is, for example, when the operation of the measurement device is started, after a predetermined time has elapsed from the previous acquisition, or the like.

As in the present modification example, the reference light intensity may be obtained by irradiating the reference reflective surface 28a, of which the reflectance is known, with the light L1, and acquiring the light intensity of the reflected light from the reference reflective surface 28a. Accordingly, the intensity of the incident light can be known with high accuracy. Therefore, in this case, for example, even when the light intensity of the light L1 emitted from the light-emitting portion 211, namely, the intensity of the incident light varies over time, the refractive index distribution can be measured with high accuracy.

As in the present modification example, the optical unit 21A of the light intensity acquisition unit 20 may include the reference reflective surface 28a and the mechanism for changing the position of the reference reflective surface 28a between a position on the optical path of the light L1 and a position avoiding the optical path. In this case, the above-described reference light intensity can be easily acquired.

[Second modification example]

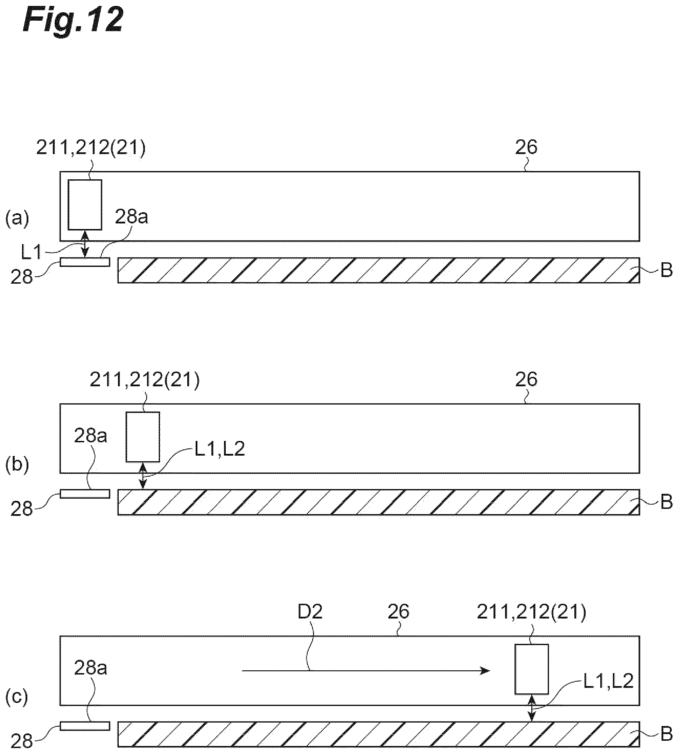

Parts (a), (b), and (c) in FIG. 12 are front views illustrating a part of a configuration of a light intensity acquisition unit as a second modification example. In these figures as well, the polymer film B is illustrated in a cross section cut along a plane perpendicular to the conveying direction D1. The light intensity acquisition unit further includes the reflective plate 28 in addition to the configuration of the light intensity acquisition unit 20 of the embodiment. A configuration of the reflective plate 28 is the same as that of the first modification example, except for the following respects. The reflective plate 28 of the present modification example is provided independently of the optical unit 21, and is disposed side by side with the polymer film B in the direction D2. Furthermore, the linear guide 26 is configured to scan the optical unit 21 over a range from above the polymer film B to above the reflective plate 28. Part (a) in FIG. 12 illustrates a case where the optical unit 21 is located above the reference reflective surface 28a. In this case, the light L1 emitted from the light-emitting portion 211 is reflected by the reference reflective surface 28a. Reflected light from the reference reflective surface 28a is incident on the light incident portion 212. Then, the light intensity of the reflected light for each wavelength is detected by the spectral detection unit 23, so that the arithmetic processor 30 can obtain the reference light intensity, which is to be used when the reflectance R of the polymer film B is calculated, for each wavelength. Part (b) of FIG. 12 illustrates a case where the optical unit 21 is located above the polymer film B. In this case, the light L1 emitted from the light-emitting portion 211 is reflected by the polymer film B. The reflected light L2 from the polymer film B is incident on the light incident portion 212. Then, the light intensity of the reflected light L2 for each wavelength is detected by the spectral detection unit 23. The arithmetic processor 30 calculates the refractive index n of the polymer film B based on these light intensities. Then, as illustrated in part (c) of FIG. 12, the linear guide 26 scans the optical unit 21 along the direction D2 of the polymer film B. At this time, the arithmetic processor 30 can obtain the refractive index distribution by repeating the calculation of the refractive index n. In the present modification example as well, the acquisition of the reference light intensity using the reference reflective surface 28a (part (a) in FIG. 12) is repeatedly performed at the above-described predetermined timing.

[Third modification example]

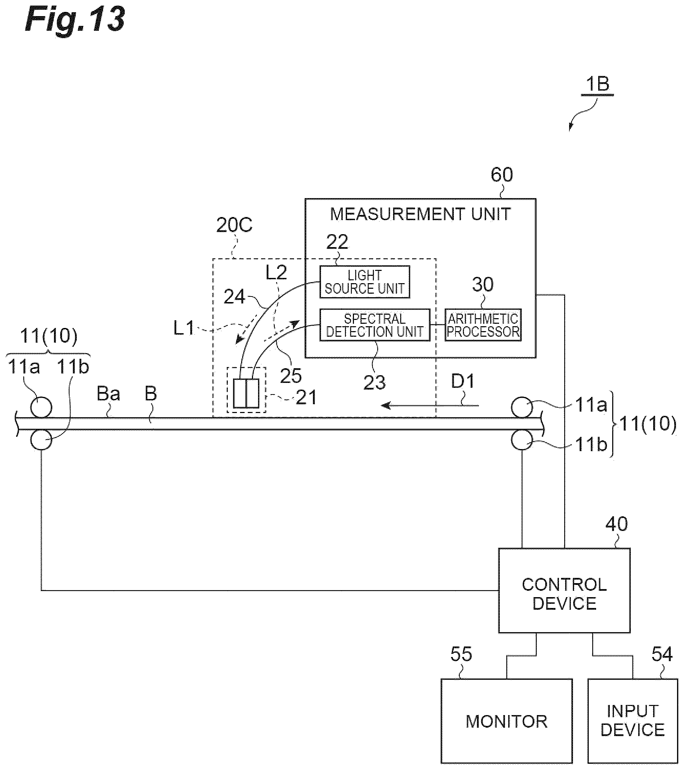

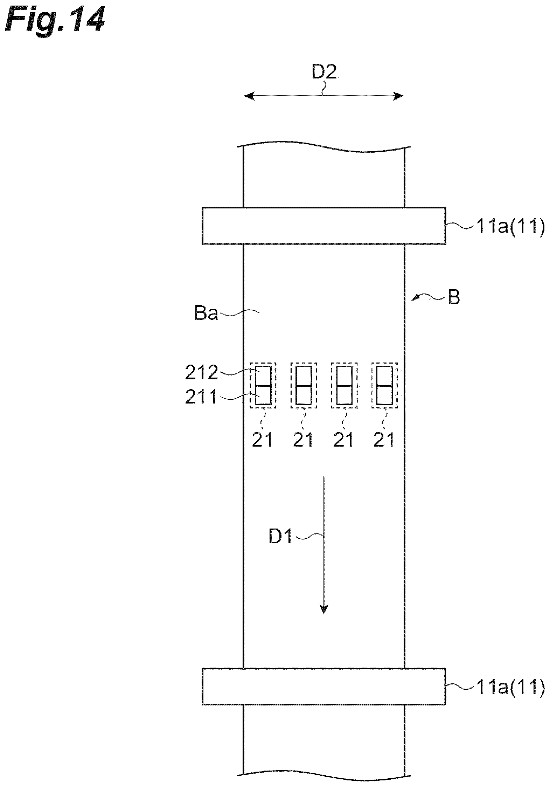

FIG. 13 is a view schematically illustrating a configuration of a measurement device 1B according to a third modification example. The measurement device 1B differs from the measurement device 1A of the embodiment in that the measurement device 1B includes a light intensity acquisition unit 20C instead of the light intensity acquisition unit 20 of the embodiment. The light intensity acquisition unit 20C differs from the light intensity acquisition unit 20 of the embodiment in that the light intensity acquisition unit 20C does not include the linear guide 26 and the control unit 27 and is provided with a plurality of the optical units 21, and is identical to the light intensity acquisition unit 20 of the embodiment in other respects. FIG. 14 is a plan view illustrating a plurality of the optical units 21. The plurality of (in the illustrated example, four) optical units 21 correspond to the plurality of respective spots SP (refer to FIG. 2) to be irradiated with the light L1. The plurality of optical units 21 are disposed side by side along the direction D2 intersecting the conveying direction D1. Intervals between the plurality of optical units 21 may be uniform. In the present embodiment, the light source unit 22, the spectral detection unit 23, the light guide members 24 and 25, and the arithmetic processor 30 are provided to correspond to each of the optical units 21.

As in the present modification example, the light intensity acquisition unit 20C may include the plurality of optical units 21 corresponding to the plurality of respective spots SP. For example, with such a configuration as well, the plurality of spots SP can be irradiated with the light L1, and the light intensity of the reflected light L2 from each of the plurality of spots SP irradiated with the light L1 can be acquired. Then, the refractive index distribution and the film thickness distribution in the direction D2 intersecting the conveying direction D1 can be measured.

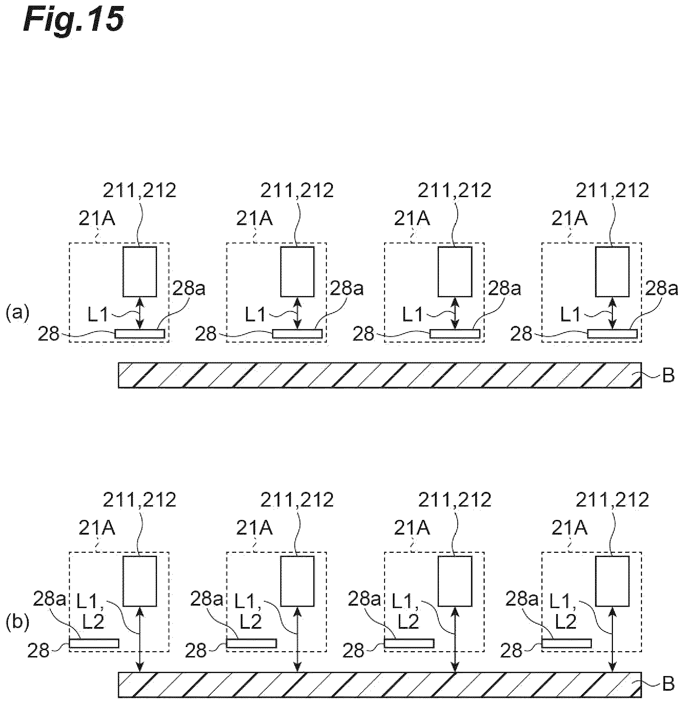

The light intensity acquisition unit 20C may include a plurality of the optical units 21A of the first modification example instead of the plurality of optical units 21. Parts (a) and (b) in FIG. 15 are front views illustrating a mode in which the plurality of (in the illustrated example, four) optical units 21A are disposed side by side along the direction D2. Part (a) in FIG. 15 illustrates a case where the reference reflective surface 28a is located on the optical path of the light L1 emitted from the light-emitting portion 211. Part (b) in FIG. 15 illustrates a case where the reference reflective surface 28a is located to avoid the optical path of the light L1 emitted from the light-emitting portion 211. The operation of the light intensity acquisition unit 20C in these cases is the same as the operation of the light intensity acquisition unit in the first modification example.

The refractive index distribution measurement device, the film thickness distribution measurement device, the refractive index distribution measurement method, the film thickness distribution measurement method according to the present disclosure are not limited to the embodiment and each modification example described above, and various other modifications can be made. For example, the first to third modification examples described above may be combined with each other depending on the required purpose and effect.

The principles of the present invention have been illustrated and described in the preferred embodiments; however, those skilled in the art will recognize that the present invention can be changed in disposition and details without departing from such principles. The present invention is not limited to the specific configurations disclosed in the present embodiment. Therefore, rights to all modifications and changes deriving from the claims and the scope of the concept thereof are claimed.

**Reference Signs List**

1A, 1B: measurement device, 10: conveyor, 11: roller pair, 11a, 11b: roller, 20, 20C: light intensity acquisition unit, 21, 21A: optical unit, 22: light source unit, 23: spectral detection unit, 24, 25: light guide member, 26: linear guide, 27: control unit, 28: reflective plate, 28a: reference reflective surface, 30: arithmetic processor, 31: processor, 32: ROM, 34: auxiliary storage device, 40: control device, 41: conveyance control unit, 42: scanning control unit, 43: detection control unit, 54: input device, 55: monitor, 60: measurement unit, 211: light-emitting portion, 212: light incident portion, B: polymer film, Ba: major surface, Bb: surface, d: film thickness, D1: conveying direction, D2: direction, L1: light, L2: reflected light, SP: spot.

**Claims**

1. A refractive index distribution measurement device comprising:

   a conveyor that conveys a polymer film in a first direction;
   a light intensity acquisition unit that irradiates a plurality of spots on the polymer film being conveyed with light, the plurality of spots being arranged in a second direction intersecting the first direction, and that acquires a light intensity of reflected light from each of the plurality of spots irradiated with the light; and
   an arithmetic processor that calculates a reflectance at each of the plurality of spots from the light intensity of the reflected light acquired by the light intensity acquisition unit, and that obtains a refractive index distribution of the polymer film in the second direction based on the reflectance.

2. The refractive index distribution measurement device according to claim 1,
   wherein the arithmetic processor calculates the reflectance at each of the plurality of spots based on a ratio of the light intensity of the reflected light from each of the plurality of spots to a reference light intensity obtained by irradiating a reference reflective surface, of which a reflectance is known, with the light from the light intensity acquisition unit, and acquiring a light intensity of reflected light from the reference reflective surface in the light intensity acquisition unit.

3. The refractive index distribution measurement device according to claim 2,
   wherein the light intensity acquisition unit includes the reference reflective surface, and a mechanism for changing a position of the reference reflective surface between a position on an optical path of the light emitted from the light intensity acquisition unit and a position avoiding the optical path.

4. The refractive index distribution measurement device according to any one of claims 1 to 3,

   wherein the light intensity acquisition unit includes a plurality of optical units respectively corresponding to the plurality of spots, and
   each of the plurality of optical units includes a light-emitting portion that emits the light and a light incident portion that receives the reflected light.

**5.** The refractive index distribution measurement device according to any one of claims 1 to 3, wherein the light intensity acquisition unit includes an optical unit including a light-emitting portion that emits the light, and a light incident portion that receives the reflected light, and a scanning portion that scans the optical unit in a direction intersecting the first direction.

**6.** The refractive index distribution measurement device according to any one of claims 1 to 5, wherein the arithmetic processor obtains the refractive index distribution further based on an extinction coefficient of the polymer film.

**7.** The refractive index distribution measurement device according to any one of claims 1 to 6, wherein a wavelength band of the light is in a visible wavelength range, or is a wavelength band ranging from the visible wavelength range to a near-infrared wavelength range.

**8.** The refractive index distribution measurement device according to any one of claims 1 to 6, wherein a wavelength band of the light includes a near-infrared wavelength range.

**9.** A film thickness distribution measurement device comprising:

the refractive index distribution measurement device according to any one of claims 1 to 8, wherein the arithmetic processor further obtains a film thickness distribution of the polymer film in the second direction based on the light intensity of the reflected light at each of the plurality of spots acquired by the light intensity acquisition unit and on the refractive index distribution.

**10.** A refractive index distribution measurement method comprising:

a step of starting to convey a polymer film in a first direction;
a step of irradiating a plurality of spots on the polymer film being conveyed with light, the plurality of spots being arranged in a second direction intersecting the first direction, and acquiring a light intensity of reflected light from each of the plurality of spots irradiated with the light; and
a step of calculating a reflectance at each of the plurality of spots from the light intensity of the reflected light acquired in the step of acquiring, and obtaining a refractive index distribution of the polymer film in the second direction based on the reflectance.

**11.** The refractive index distribution measurement method according to claim 10, wherein in the step of obtaining the refractive index distribution, the reflectance at each of the plurality of spots is calculated based on a ratio of the light intensity of the reflected light from each of the plurality of spots to a reference light intensity obtained by irradiating a reference reflective surface, of which a reflectance is known, with the light, and acquiring a light intensity of reflected light from the reference reflective surface.

**12.** The refractive index distribution measurement method according to claim 10 or 11, wherein in the step of obtaining the refractive index distribution, the refractive index distribution is obtained further based on an extinction coefficient of the polymer film.

**13.** The refractive index distribution measurement method according to any one of claims 10 to 12, wherein a wavelength band of the light is in a visible wavelength range, or is a wavelength band ranging from the visible wavelength range to a near-infrared wavelength range.

**14.** The refractive index distribution measurement method according to any one of claims 10 to 12, wherein a wavelength band of the light includes a near-infrared wavelength range.

**15.** A film thickness distribution measurement method comprising:

the refractive index distribution measurement method according to any one of claims 10 to 14; and
a step of obtaining a film thickness distribution of the polymer film in the second direction based on the light intensity of the reflected light at each of the plurality of spots acquired in the step of acquiring the light intensity and on the refractive index distribution obtained in the step of obtaining the refractive index distribution.

# Fig.1

1A

60

MEASUREMENT UNIT

20

22

L2

24

LIGHT SOURCE UNIT

30

L1

SPECTRAL DETECTION UNIT

ARITHMETIC PROCESSOR

11(10)

11a  11b

26

25

23

D1

Ba  B

21

11a

11(10)

11b

27  CONTROL UNIT

40

CONTROL DEVICE

55

54

MONITOR

INPUT DEVICE

*Fig.2*

# Fig.3

## Fig.4

(a)

(b)

# Fig.5

(a)

(b)

# Fig.6

# Fig.7

(a) INTENSITY

WAVELENGTH

(b) INTENSITY

WAVELENGTH

(c) INTENSITY

WAVELENGTH

FILM
THICKNESS
IS SMALL

FILM
THICKNESS
IS LARGE

# Fig.8

30

31 — CPU

32 — ROM

33 — RAM

34 — AUXILIARY STORAGE DEVICE

*Fig.9*

40

CONTROL DEVICE

| CONVEYANCE CONTROL UNIT | 41 |

| SCANNING CONTROL UNIT | 42 |

| DETECTION CONTROL UNIT | 43 |

...

**Fig.10**

```
           ┌─────────────┐
           │    START    │
           └──────┬──────┘
                  │
    ┌─────────────────────────────┐
    │      START CONVEYANCE       │──── ST1
    └─────────────┬───────────────┘
                  │
    ┌─────────────────────────────┐
    │     DETECT REFLECTED LIGHT  │──── ST2
    │     AT PLURALITY OF SPOTS   │
    └─────────────┬───────────────┘
                  │
    ┌─────────────────────────────┐
    │    CALCULATE REFRACTIVE     │──── ST3
    │     INDEX DISTRIBUTION      │
    └─────────────┬───────────────┘
                  │
    ┌─────────────────────────────┐
    │   CALCULATE FILM THICKNESS  │──── ST4
    │        DISTRIBUTION         │
    └─────────────┬───────────────┘
                  │
           ┌─────────────┐
           │     END     │
           └─────────────┘
```

# Fig.11

(a)

(b)

(c)

**Fig.12**

(a)

211,212(21) 26

28a
L1
28 B

(b)

211,212(21) 26

28a
L1,L2
28 B

(c)

D2 26 211,212(21)

28a
L1,L2
28 B

*Fig.13*

*Fig.14*

# Fig.15

(a)

211,212   211,212   211,212   211,212
21A       21A       21A       21A

28a       28a       28a       28a
L1        L1        L1        L1
28        28        28        28

B

(b)

211,212   211,212   211,212   211,212
21A       21A       21A       21A

28a       28a       28a       28a
L1,       L1,       L1,       L1,
L2        L2        L2        L2
28        28        28        28

B

## INTERNATIONAL SEARCH REPORT

International application No.

**PCT/JP2023/007615**

### A. CLASSIFICATION OF SUBJECT MATTER

***G01N 21/41***(2006.01)i; ***G01B 11/04***(2006.01)i

FI: G01N21/41 Z; G01B11/04 101Z

According to International Patent Classification (IPC) or to both national classification and IPC

### B. FIELDS SEARCHED

Minimum documentation searched (classification system followed by classification symbols)

G01N21/00-G01N21/61; G01J4/00-G01J4/04; G01J7/00-G01J9/04; G01B11/00-G01B11/30; G01N21/84-G01N21/958; C08J5/00-C08J5/02; G02B5/20-G02B5/28; G02B5/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2006-175616 A (NIPPON ZEON CO., LTD.) 06 July 2006 (2006-07-06) paragraphs [0014], [0017], [0018], [0021], [0028]-[0035], fig. 1-4 | 1-2, 4-15 |
| A | | 3 |
| Y | JP 7-159131 A (NKK CORP.) 23 June 1995 (1995-06-23) paragraphs [0089]-[0092], fig. 12, 13 | 1-2, 4-15 |
| Y | JP 2015-141176 A (HAMAMATSU PHOTONICS KK) 03 August 2015 (2015-08-03) paragraphs [0031]-[0069], fig. 1-10 | 1-2, 4-15 |
| A | JP 2005-351790 A (TORAY IND., INC.) 22 December 2005 (2005-12-22) entire text, all drawings | 1-15 |
| A | CN 210897492 U (JINHUA JIN QIU ENVIRONMENTAL PROTECTION WATER TREATMENT CO., LTD.) 30 June 2020 (2020-06-30) entire text, all drawings | 1-15 |

☐ Further documents are listed in the continuation of Box C.　　☑ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **18 April 2023** | **09 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2023/007615**

| Patent document cited in search report | | | Publication date (day/month/year) | Patent family member(s) | | | Publication date (day/month/year) |
|---|---|---|---|---|---|---|---|
| JP | 2006-175616 | A | 06 July 2006 | (Family: none) | | | |
| JP | 7-159131 | A | 23 June 1995 | (Family: none) | | | |
| JP | 2015-141176 | A | 03 August 2015 | US | 2016/0349038 | A1 | |
| | | | | paragraphs [0037]-[0074], fig. 1-10 | | | |
| | | | | WO | 2015/114895 | A1 | |
| | | | | DE | 112014006304 | T | |
| | | | | TW | 201530091 | A | |
| | | | | CN | 105940282 | A | |
| | | | | KR | 10-2016-0114080 | A | |
| JP | 2005-351790 | A | 22 December 2005 | (Family: none) | | | |
| CN | 210897492 | U | 30 June 2020 | (Family: none) | | | |

Form PCT/ISA/210 (patent family annex) (January 2015)

28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015141176 A
- WO 2011045967 A